# EUROPEAN PATENT APPLICATION

(11) **EP 4 112 600 A1**
(43) Date of publication of application: **04.01.2023**
(21) Application number: 21183581.4
(22) Date of filing: 03.07.2021
(51) Int. Cl.: C07D 205/04, C07D 209/08, C07D 209/12, C07D 209/20, C07D 209/88, C07D 209/96, C07D 213/68, C07D 231/12, C07D 231/16, C07D 231/38, C07D 233/64, C07D 249/10, C07D 263/24, C07D 265/30, C07D 295/192, C07D 333/72, C07D 339/08, C07D 401/12, C07D 405/04, C07D 471/04

(54) **VINYL THIANTHRENIUM COMPOUND, PROCESS FOR ITS PREPARATION AND ITS USE FOR TRANSFERRING A VINYL GROUP**

(71) Applicant: Studiengesellschaft Kohle mbH, 45470 Mülheim (DE)
(72) Inventor: RITTER, Tobias, 45470 Muelheim an der Ruhr (DE); JULIA HERNANDEZ, Fabio, 45470 Mülheim an der Ruhr (DE)

(57) **Abstract**

The present application relates to vinyl thianthrenium compounds of Formula (I) such as vinyl-TT⁺ (1), a process for preparing the same and the use thereof for vinylating organic compounds.

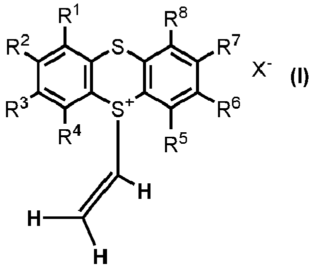

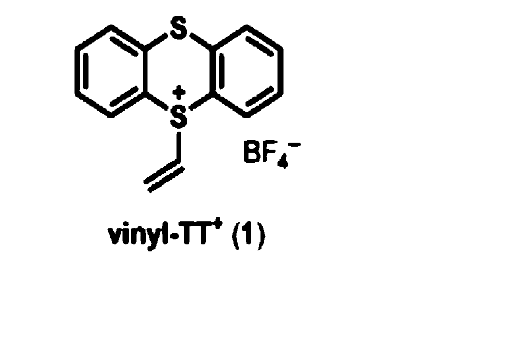

## Description

The present invention refers a novel vinyl thianthrenium compound, in the following referred to as vinyl-TT⁺X⁻, a process for preparing the same and the use thereof for vinylating organic compounds.

Due to the rich chemistry of alkenes, the presence of a terminal alkenyl (vinyl, C₂H₃) substituent in a given structure enables a myriad of opportunities for diversification and elaboration via epoxidation, dihydroxylation, aziridination, hydroboration, carbofunctionalization, Heck-type arylation, hydroamination and metathesis, among others However, the introduction of a vinyl group as a C-2 building block is currently difficult due to the lack of suitable reagents with the desired reactivity profile. Here, the inventors report the new reagent vinyl thianthrenium compound (vinyl-TT⁺X⁻, **1)** that functions as a versatile reagent for different synthetic transformations. The new sulfonium salt is accessible directly from ethylene (1 atm) in a single step from commercially available material on multigram scale, and can be isolated in pure form by simple precipitation. It is a bench-stable, non-hygroscopic, crystalline, free-flowing solid that can be stored at room temperature in air without signs of decomposition for at least eight months. Despite its high stability, vinyl-TT⁺ displays a rich reactivity profile and has been successfully implemented in several polar reactions as well as in palladium-catalysed cross-coupling reactions, which distinguishes it from all other vinylating reagents reported to date.

Vinylated compounds can be generated by elimination from the corresponding (pseudo)halides but the harsh reaction conditions required prevent the use of this route for structurally complex substrates. Alternatively, synthesis from aldehydes via Wittig olefination with methylene phosphonium ylide as C1 synthon requires that one carbon of the vinyl group is already present in the starting material. The development of transition metal cross-coupling reactions in the last decades, particularly those based on palladium, has allowed researchers to reliably construct C-Csp² bonds. However, in contrast to the widespread use of other substituted alkenyl derivatives, the use of vinyl halides as electrophiles (e.g. vinyl bromide) in cross-couplings is challenging due to the difficulty in handling of these gaseous compounds that are acutely toxic and carcinogenic. Numerous nucleophilic vinyl-[M] reagents ([M]= SnBu₃, SiMe₃, B(OR)₂, etc) have been developed over the years, but most of them are prepared in several steps from vinyl bromide itself, display high toxicity, low stability, or are poorly reactive. Moreover, while significant advances have been accomplished with vinyl nucleophiles, the development of electrophilic derivatives that can effectively display the reactivity profile of vinyl halides is significantly less accomplished, and none of them are suitable as Michael acceptors for the direct polar addition of nucleophiles.

Jimenez, Mukaiyama and Aggarwal have developed the use of vinyl diphenylsulfonium salts as a 1,2-ethane dication synthon. This hygroscopic oil, prepared in three steps from bromoethanol, displays some practicality issues and is often generated in situ from its precursor bromoethyl diphenylsulfonium triflate. Over the past two decades, Aggarwal and others have reported a series of elegant transformations applying this reagent to the synthesis of (hetero)cycles. However, neither the reagent nor its precursors have ever been reported as suitable electrophiles in transition-metal cross-coupling reactions due to their fundamental reactivity profile (vide infra).

The group of the present inventors recently reported the C-H thianthrenation of olefins to access alkenyl thianthrenium salts (Angew. Chem. Int. Ed. 2020, 59, 5616 -5620). Under those reaction conditions, a highly electrophilic thianthrenium-based intermediate (i.e. thianthrene dication) is formed, which undergoes a formal [4+2] cycloaddition to the alkene substrate and, after basic workup, affords the alkenyl sulfonium product. With the aim of providing a bench-stable and versatile reagent that could be obtained directly from the feedstock gas ethylene (annual production over 100 million tons), the inventors succeeded to prepare vinyl thianthrenium salts from thianthrene-S-oxide.

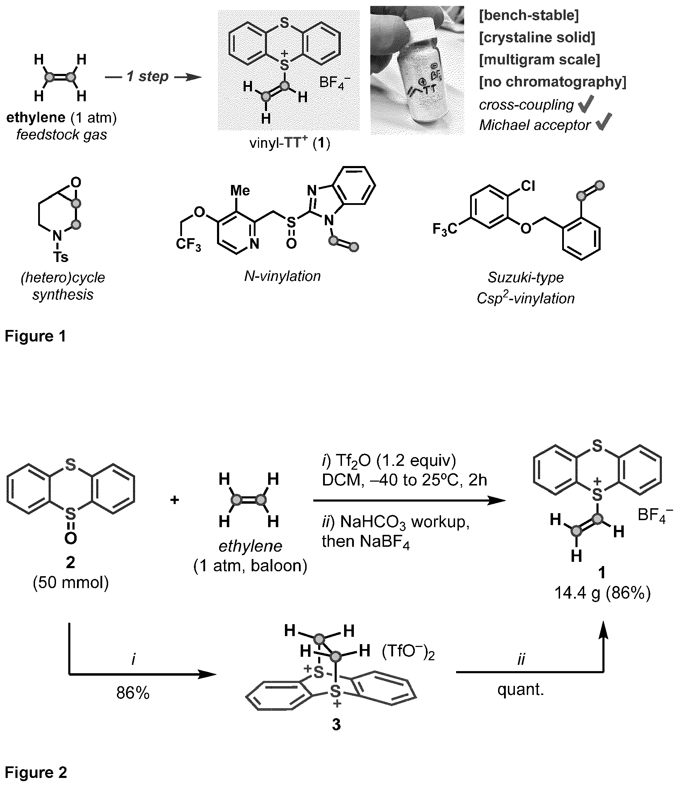

Optimization of the protocol resulted in the preparation of vinyl thianthrenium tetrafluoroborate **(1)** on multigram-scale (50 mmol) by reaction of thianthrene-S-oxide **(2)** with triflic anhydride in an ethylene atmosphere (1 atm, using a balloon) in 86% yield (Figure 2A). The isolation of **1** as a crystalline off-white solid can be carried out after workup by simple precipitation, to afford analytically pure thianthrenium salt without the need for further purification. The salt **1** is a non-hygroscopic solid that can be stored in the presence of air and moisture without signs of decomposition for at least eight months, which makes it a practical and easy-to-handle reagent. Differential scanning calorimetry/thermogravimetric analysis (DSC-TGA) reveals that **1** does not decompose at temperatures lower than 280 °C, underscoring a desirable safety profile. A solid sample of **1** heated at 200°C for 5 minutes shows no signs of decomposition as judged by ¹H NMR spectroscopy (Figure S3). In contrast, attempts of implementing this protocol for the synthesis of derivatives of other sulfoxides such as diphenyl- or dibenzothiophene-S-oxide from ethylene were unsuccessful. The structural features of thianthrene that allow the formation of a [4+2] adduct with ethylene seem crucial for a productive reaction.

Next, the inventors succeeded to implement 1 in new reactions to effectively transfer the vinyl moiety to hydrocarbons including nucleophilic nitrogen.

Thus the present invention is directed to a thianthrene compound of the Formula (I): 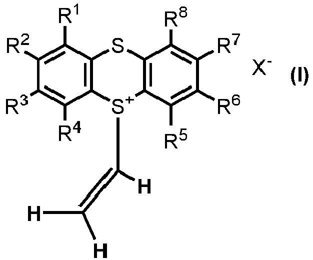 wherein R¹ to R⁸ may be the same or different and are each selected from hydrogen, halogen, -OR⁰ wherein R⁰ is hydrogen or a C₁ to C₆ alkyl group, which may be substituted by at least one halogen, -NR^{N1}R^{N2} wherein R^{N1} and R^{N2} are the same or different and are each hydrogen or each a C₁ to C₆ alkyl group, which may be substituted by at least one halogen, or a C₁ to C₆ alkyl group, which may be substituted by at least one halogen, and wherein X⁻ is an anion, selected from F⁻, Cl⁻, triflate⁻ , BF₄⁻, SbF₆⁻, PF₆⁻, BAr₄⁻, TsO⁻, MsO⁻, ClO₄⁻, 0.5 SO₄²⁻, or NO₃⁻ with the proviso that the compound of Formula (I) with R₁ to R₈ being hydrogen and X being ClO₄⁻ is excluded. The solubility of the salts cover a wide range of organic solvents and water, which can be tuned with an appropriate selection of the counterion.

In a particular embodiment, the present invention refers to a thianthrene compound of the Formula (I) as defined before wherein, in Formula (I), R¹ to R⁸ may be the same or different and are each selected from hydrogen, Cl or F and X⁻ is an anion as defined before, preferably triflate or BF₄⁻ with the proviso that the compound of Formula (I) with R₁ to R₈ being hydrogen and X being ClO₄⁻ is excluded..

In another embodiment, the present invention refers to a thianthrene compound of the Formula (I) as clamed in claim 1 wherein, in Formula (I), R¹ to R⁸ are each hydrogen, and X⁻ is an anion as defined in claim 1, preferably triflate or BF₄⁻ with the proviso that the compound of Formula (I) with R₁ to R₈ being hydrogen and X being ClO₄⁻ is excluded..

The inventive compound of the Formula (I) may be prepared in a process whereby, in a first step, a thianthrene-S-oxide derivative of the Formula (II) is reacted with ethylene in a closed reaction vessel, at a pressure of at least one atm, in an organic solvent in the presence of triflic acid anhydride or any other combination of acylating reagents such as trifluoroacetic anhydride in the presence of a Bronsted/Lewis acid. Acyl halides and anhydrides of carboxylic acids may be used as acylating agents. In a second step the obtained reaction mixture is treated with an aqueous basic solution and the obtained reaction product is finally treated with an alkali salt, preferably in aqueous solution, whereby a thianthrenium compound of the Formula (I) is obtained: 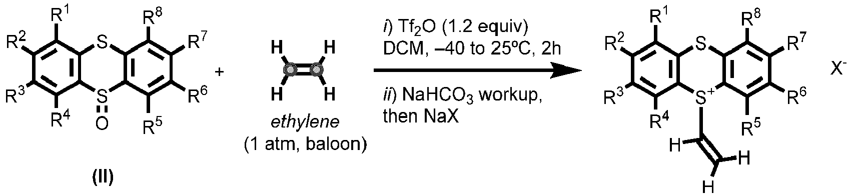 wherein R¹ to R⁸ may be the same or different and are each selected from hydrogen, halogen, -OR⁰ wherein R⁰ is hydrogen or a C₁ to C₆ alkyl group, which may be substituted by at least one halogen, -NR^{N1}R^{N2} wherein R^{N1} and R^{N2} are the same or different and are each hydrogen or each a C₁ to C₆ alkyl group, which may be substituted by at least one halogen, or a C₁ to C₆ alkyl group, which may be substituted by at least one halogen,
and wherein X⁻ is an anion, selected from F⁻, Cl⁻, triflate- , BF₄⁻, SbF₆⁻, PF₆⁻, BAr₄⁻, TsO⁻, MsO⁻, ClO₄⁻, 0.5 SO₄²⁻, or NO₃.

The present invention is also directed to the use of the inventive vinyl thianthrenium compound of the Formula (I) wherein R¹ to R⁸ may be the same or different and are each selected from hydrogen, halogen, or a C₁ to C₆ alkyl group, which may be substituted by at least one halogen, and wherein X⁻ is an anion, selected from F⁻, Cl⁻, triflate- , BF₄⁻, SbF₆⁻, PF₆⁻, ClO₄⁻, 0.5 SO₄²⁻, or NO₃⁻.and X⁻ is an anion as defined before as a transfer agent for transferring a vinyl group to a hydrocarbon compound, in particular to an aliphatic hydrocarbon, to an aromatic hydrocarbon, to a heteroaromatic hydrocarbon, to a hydrocarbon bearing at least one boric acid group or to a hydrocarbon bearing at least one nucleophilic heteroatom. In the latter case if the hydrocarbon is bearing two nucleophilic heteroatoms, a ring system with the nucleophilic heteroatoms may be formed. As an example, a 1-hydroxy-ω-amino-C_{X}H_{Y}-hydrocarbon will react with the inventive compound of formula (I) to form a hydrocarbon ring system with a -O-C₂H₄-NH-group in the ring system so that a C₂H₄-unit is inserted between the heteroatoms.

In the inventive process for preparing the inventive Vinyl-TT⁺X⁻ or the use thereof for vinyl transfer, the choice of the organic solvent is not critical as long as it is an aprotic organic solvent selected from acetonitrile, other nitriles, chlorinated hydrocarbons, or other aprotic solvents, or mixtures thereof. The reaction conditions are also not critical and the reaction is usually carried out at a temperature between -78°C and 50°C, preferably -40°C to 30°C, under an ethylene atmosphere under a pressure of at least one atm for the first step.

In the inventive process for transferring the vinyl group, the aromatic hydrocarbon or heteroaromatic hydrocarbon may be a monocyclic or polycyclic, aromatic or heteroaromatic hydrocarbon having 5 to 22 carbon atoms, which may be unsubstituted or substituted by one of more substituents selected from saturated or unsaturated, straight chain or branched aliphatic hydrocarbons having 1 to 20 carbon atoms, aromatic or heteroaromatic hydrocarbons having 5 to 22 carbon atoms, heterosubstituents, or which may be part of a cyclic hydrocarbon ring system (carbocyclic) with 5 to 30 carbon atoms and/or heteroatoms. The definition for said aliphatic hydrocarbons may include one or more heteroatoms such O, N, S in the hydrocarbon chain.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The term "aliphatic" includes both saturated and unsaturated, straight chain (*i.e.,* unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and acyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (acyclic, substituted, unsubstituted, branched or unbranched) having 1-6 carbon atoms.

As used herein, "alkyl" refers to a radical of a straight-chain, branched or cyclic saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (e.g., -CH₃). In certain embodiments, the alkyl group is a substituted C₁₋₁₀ alkyl.

"Aryl" refers to a radical of a monocyclic or polycyclic (*e.g*., bicyclic or tricyclic) 4n+2 aromatic ring system (*e.g*., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g*., phenyl). In some embodiments, an aryl group has ten ring carbon atoms ("C₁₀ aryl"; *e.g*., naphthyl such as 1-naphthyl and 2-naphthyl). In some embodiments, an aryl group has fourteen ring carbon atoms ("C₁₄ aryl"; *e.g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" refers to a radical of a 5-14 membered monocyclic or bicyclic 4n+2 aromatic ring system (e.g., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, *i.e.,* either the ring bearing a heteroatom (*e.g.,* 2-indolyl) or the ring that does not contain a heteroatom (*e.g.*, 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl and refers to an optionally substituted alkyl group substituted by an optionally substituted heteroaryl group.

"Unsaturated" or "partially unsaturated" refers to a group that includes at least one double or triple bond. A "partially unsaturated" ring system is further intended to encompass rings

having multiple sites of unsaturation, but is not intended to include aromatic groups (e.g., aryl or heteroaryl groups) as herein defined. Likewise, "saturated" refers to a group that does not contain a double or triple bond, *i.e.,* contains all single bonds.

Alkyl, alkenyl, alkynyl, carbocyclyl, heterocyclyl, aryl, and heteroaryl groups, which are divalent bridging groups, are further referred to using the suffix -ene, e.g., alkylene, alkenylene, alkynylene, carbocyclylene, heterocyclylene, arylene, and heteroarylene.

An atom, moiety, or group described herein may be unsubstituted or substituted, as valency permits, unless otherwise provided expressly. The term "optionally substituted" refers to substituted or unsubstituted.

Alkyl, alkenyl, alkynyl, carbocyclyl, heterocyclyl, aryl, and heteroaryl groups are optionally substituted (e.g., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. For purposes of this invention, heteroatoms such as nitrogen may have hydrogen substituents and/or any suitable substituent as described herein which satisfy the valencies of the heteroatoms and results in the formation of a stable moiety. In certain embodiments, the substituent is a carbon atom substituent. In certain embodiments, the substituent is a nitrogen atom substituent. In certain embodiments, the substituent is an oxygen atom substituent. In certain embodiments, the substituent is a sulfur atom substituent.

Exemplary substituents include, but are not limited to, halogen, -CN, -NO₂, -N₃, -SO₂H, - SO₃H, -OH, -O-alkyl, -N-dialkyl, -SH, -S.alkyl, -C(=O)alkyl,-CO₂H, -CHO.

"Halo" or "halogen" refers to fluorine (fluoro, -F), chlorine (chloro, -CI), bromine (bromo, - Br), or iodine (iodo, -I).

"Acyl" refers to a moiety selected from the group consisting of -C(=O)R^{aa},-CHO, -CO₂R^{aa}, -C(=O)N(R^{bb})₂, -C(=NR^{bb})R^{aa}, -C(=NR^{bb})OR^{aa}, -C(=NR^{bb})N(R^{bb})₂, -C(=0)NR^{bb}SO₂R^{aa}, - C(=S)N(R^{bb})₂, -C(=O)SR^{aa}, or -C(=S)SR^{aa}, wherein R^{aa} and R^{bb} are as defined below.

Each instance of R^{aa} is, independently, selected from C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, and each instance of R^{bb} is, independently, selected from hydrogen, -OH, -OR^{aa}, -N(R^{cc})₂, -CN, -C(=O)R^{aa}, -C(=O)N(R^{cc})₂, -CO₂R^{aa}, -SO2R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, - C(=S)N(R^{cc})₂, -C(=O)SR^{cc}, -C(=S)SR^{cc}, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring.

The term "catalysis," "catalyze," or "catalytic" refers to the increase in rate of a reaction due to the participation of a substance called a "catalyst." In certain embodiments, the amount and nature of a catalyst remains essentially unchanged during a reaction. In certain embodiments, a catalyst is regenerated, or the nature of a catalyst is essentially restored after a reaction. A catalyst may participate in multiple chemical transformations. The effect of a catalyst may vary due to the presence of other substances known as inhibitors or poisons (which reduce the catalytic activity) or promoters (which increase the activity). Catalyzed reactions have a lower activation energy (rate-limiting free energy of activation) than the corresponding uncatalyzed reaction, resulting in a higher reaction rate at the same temperature. Catalysts may affect the reaction environment favorably, or bind to the reagents to polarize bonds, or form specific intermediates that are not typically produced by a uncatalyzed reaction, or cause dissociation of reagents to reactive forms.

The invention is not intended to be limited in any manner by the above exemplary listing of substituents.

The inventors have realized that heteroatom-vinylated derivatives are useful intermediates in many total syntheses and important monomeric precursors of polymers of high relevance in material science (e.g. polyvinylcarbazole). Vinylation of N-heterocycles has been achieved in some cases by using an alkylation-elimination protocol with dibromoethane, but the harsh conditions and low yields typically obtained reduce the general application of this route.

### Experimental Part

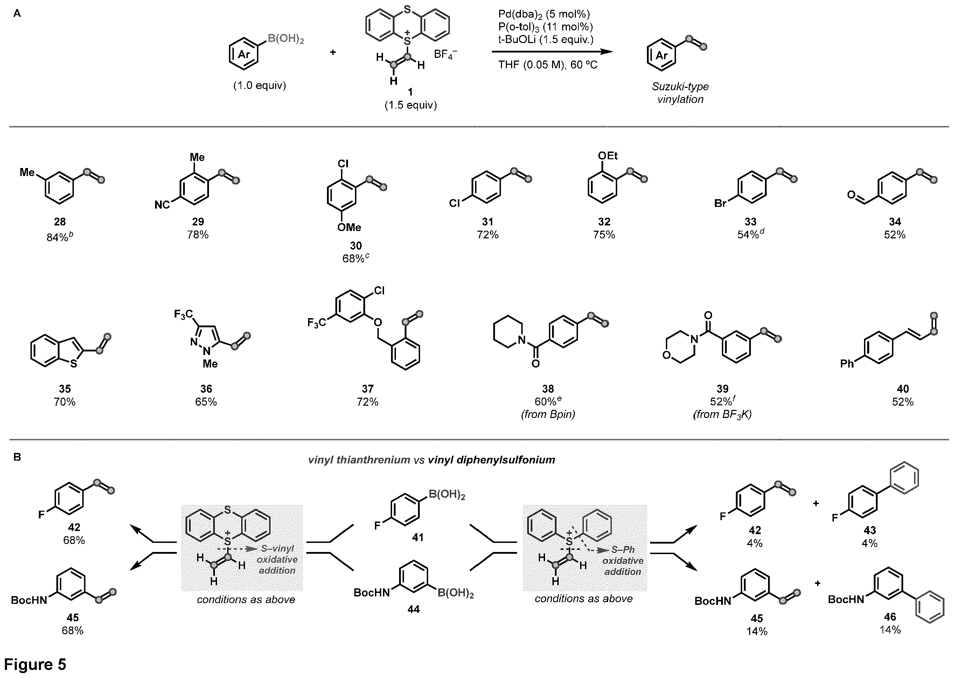

The invention is further illustrated by the attached Figures and Examples. In the Figures, the respective Figure shows:
Fig. 1: Vinyl thianthrenium salt 1 can be accessed directly from ethylene and is a versatile C-2 building block;
Fig. 2: Synthesis of 1 from ethylene and 2, proceeding through a formal [4+2] cycloadduct (3) as intermediate;
Fig. 3: Application of 1 as 1,2-bis-electrophile for the annulation of hetero- and carbocycles;
Fig. 4: Vinylation of N-heterocycles using **1**.^{[a]}
   [a] Reaction conditions: 0.300 mmol N-heterocycle, 1.7 equiv. 1, 2.0 equiv. DBU in CH₂Cl₂ (3.0 mL, c = 0.10 M), 25 °C, 3h.
   [b] DMSO instead of CH₂Cl₂ was used as solvent.
   [c] 1.2 equiv. **1** were added to a premixed solution of the N-heterocycle and DBU.
   [d] 30 min at 0 °C, followed by 2.5 h at 25 °C
Fig. 5: Suzuki-type vinylation of aromatic organoboron compounds
   (A) Scope of the transformation.
   (B) Comparison of the reactivity of **1** and vinyl diphenyl sulfonium salts.
      [a] Reaction conditions: 0.300 mmol aryl boronic acid, 1.5 equiv. **1,** 0.050 equiv. Pd(dba)₂, 0.11 equiv. P(*o*-tol)₃, 1.5 equiv. *t*-BuOLi in THF (6.0 mL, c = 0.05 M), 60 °C, 16h.
      [b] NMR yield.
      [c] K₂CO₃ instead of *t*-BuOLi was used as base.
      [d] 1.7 equiv. **1, 50** °C, 24h.
      [e] Aryl boronic pinacol ester was used. [f] Aryl trifluoroborate potassium salt was used.

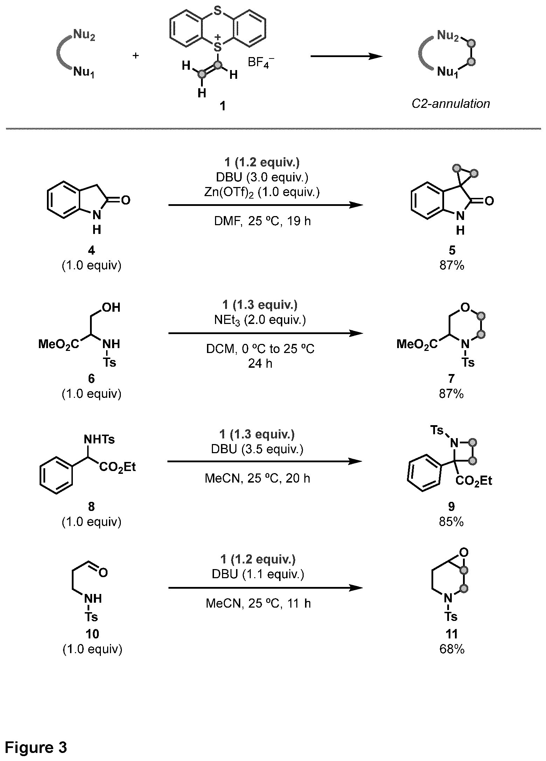

As shown in Fig. 3: To evaluate the reactivity profile of **1,** the inventors started by benchmarking the reagent in annulation reactions reported for vinyl-SPh₂(OTf) or its precursor, which proceed via sulfonium ylide intermediates.

As depicted in Figure 3, the inventors can access (hetero)cyclic motifs that are prevalent in bioactive compounds and pharmaceuticals. The selected examples include a cyclopropanation reaction (**4**→**5**), the assembly of morpholine (**6**→**7**) and azetidine (**8**→**9**) scaffolds, and a tandem N-nucleophilic addition/Corey-Chaykovsky epoxidation (**10**→**11**). In all cases, the isolated yields were comparable or superior to those obtained with vinyl-SPh₂(OTf) under the same conditions.

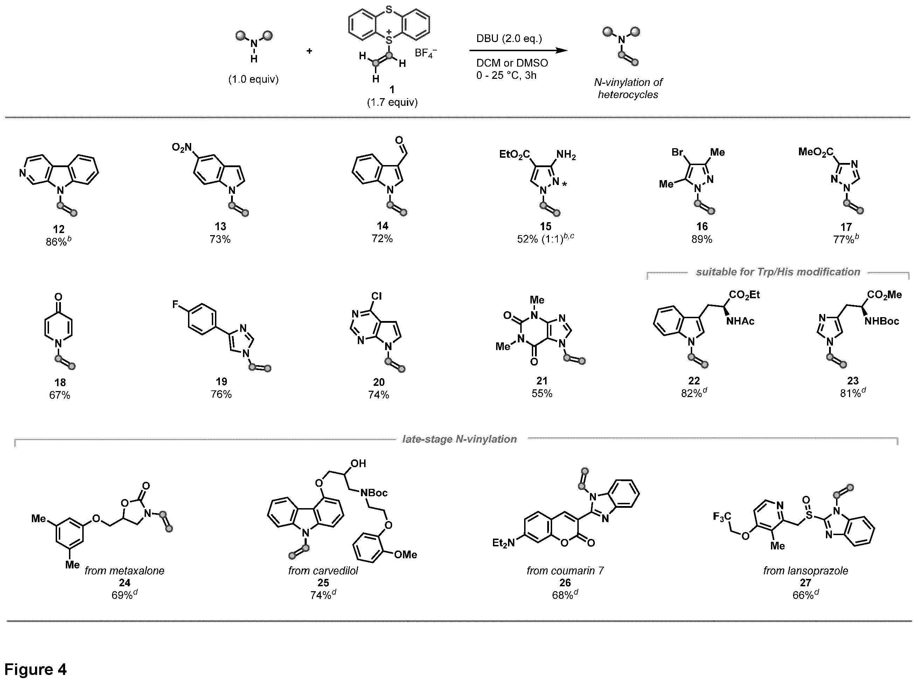

Since a general platform for N-vinylation using sulfonium salts is not yet established, the inventors optimized a simple protocol that uses **1** in the presence of a base at room temperature (Figure 4). A diverse set of N-vinylated nitrogen heterocycles can now be accessed under mild conditions with this method including azacarbazole **(12),** indole **(13-14),** imidazole **(19),** pyrazole **(15-16),** triazole **(17)** and pyridone **(18).** A broad tolerance to an array of polar groups was displayed as demonstrated by the compatibility of nitro **(13)** and aldehyde **(14)** groups, which are not tolerated using calcium carbide, or aryl halides **(16, 20)** that are reactive in S_{N}Ar and metal-catalyzed cross-coupling reactions. Although primary amines are generally not compatible, leading to aziridination products, the inventors successfully engaged **15,** containing a free amino group. Other heterocyclic scaffolds of high relevance in medicinal chemistry such as deazapurine **(20)** or theophylline **(21)** were also vinylated, as well as the amino acids tryptophan and histidine **(22-23).** Finally, the inventors explored the use of **1** for the late-stage N-vinylation of bioactive compounds and dyes. The mild conditions and fast reaction times enabled modification of the blockbuster drugs metaxalone **(24),** carvedilol **(25)** and lansoprazole **(27),** as well as the laser dye coumarin 7 **(26),** further showcasing the compatibility with functional groups such as alcohols, alkylamines, and sulfoxides.

Vinylated arenes (styrenes) are activated alkenes with widespread use in transition metal catalysis radical chemistry, and electrophilic reactions. In contrast to alkenylation, the assembly of these compounds using vinylating reagents to transfer the C₂H₃ group in metal-catalyzed cross-couplings often face several challenges such as the effective activation of the vinyl-X bond (either by oxidative addition or transmetallation), undesired Heck-type reactivity on the vinyl-[M] reagent, or further reactivity of the activated styrene-type products, which can lead to polymerization. Vinyl sulfonium salts are ideally positioned to undergo effective metal-catalyzed vinylations but no examples have been reported in the literature. In fact, only a few alkenyl sulfonium salts have been successfully engaged in cross-couplings and issues about the unselective cleavage of the different C-S bonds in these substrates have been discussed. The inventors conceived **1** as a suitable electrophilic coupling partner that could overcome the above-mentioned challenges because it should undergo fast oxidative addition due to its electropositive character (*E*_{red} = -1.13 V vs SCE).

In addition, the annulated structure of the thianthrene core should ensure that the cleavage of the C_{vinyl}-S bond would occur selectively in preference to the two C_{aryl}-S bonds, as previously demonstrated in reactions with aryl thianthrenium salts. To showcase the utility of **1** in palladium-catalyzed cross-coupling reactions, the inventors investigated the Suzuki-type vinylation of aryl boronic acids (Figure 5A). The vinylation of aryl boronic acids has been reported at T≥100 °C using vinyl bromide, acetate and tosylate as electrophiles under palladium or rhodium catalysis. Instead, the use of **1** allows this cross-coupling to be efficiently carried out at lower temperatures (60 °C), presumably due to the easier oxidative addition of C-SR₂⁺ bond in comparison with C-Br and C-O bonds. The inventors evaluated the scope of aryl boronic acids including a wide range of compounds including electron-rich **(32)** and electron-poor arenes **(29, 34)** with different functional groups and substitution patterns, bearing ortho-, meta- and para-substituents **(28-34),** including electrophilic groups that are not tolerated by Wittig olefination-based synthesis **(34, 39).** The use of electron-rich heteroarene boronic acids **(35, 36)** was also possible. The ease of oxidative addition of the C-S bond allowed the vinylation of substrates containing C-Br bonds **(33)** that are otherwise reactive in Suzuki reactions.^{[4b]} This methodology could also be extended to other organoboron compounds, such as boronic esters **(38)** and trifluoroborate salts **(39).** Alkenyl boronic acids were also found as suitable substrates, yielding valuable dienes **(40)** that can be employed for further elaboration (e.g. Diels-Alder reactions).

In contrast, the use of vinyl-SPh₂(OTf) as vinylating reagent under the same reaction conditions did not afford the desired products or resulted in <20% yield in all the cases studied (Figure 5B). For example, when using **41** as model substrate the corresponding styrene **42** was obtained in 68% yield when using reagent **1** but only 4% yield could be detected by NMR when using vinyl-SPh₂(OTf). Further analysis of the reaction mixture revealed the presence of equimolar amounts of product **43,** arising from aryl-Ph instead of aryl-vinyl bond formation, while no related product resulting from aryl-aryl coupling could be detected in the reaction with **1.** A similar outcome was observed with substrate **44.** These results underline the key benefits of the structural design of thianthrene electrophiles, effectively channelling the oxidative addition process towards the desired C-S bond.

### Materials and Methods

All reactions were carried out under an ambient atmosphere unless otherwise stated and monitored by thin-layer chromatography (TLC). Air- and moisture-sensitive manipulations were performed using standard Schlenk- and glove-box techniques under an atmosphere of argon or dinitrogen. High-resolution mass spectra were obtained using Q Exactive Plus from Thermo. Concentration under reduced pressure was performed by rotary evaporation at 25-40 °C at an appropriate pressure. When it was not removed by means of aqueous workup, DMSO was removed in a Biotage V10 evaporator. Purified compounds were further dried under vacuum (10⁻⁶ - 10⁻³ bar). Yields refer to purified and spectroscopically pure compounds, unless otherwise stated.

### Solvents

Dichloromethane, dimethylsulfoxide, acetonitrile and diethyl ether were purchased from Fisher Scientific GmbH and used as received. Anhydrous solvents were obtained from Phoenix Solvent Drying Systems. All deuterated solvents were purchased from Euriso-Top.

### Chromatography

Thin layer chromatography (TLC) was performed using EMD TLC plates pre-coated with 250 µm thickness silica gel 60 F₂₅₄ plates and visualized by fluorescence quenching under UV light and KMnO₄ stain. Flash column chromatography was performed using silica gel (40-63 µm particle size) purchased from Geduran^{®}.

### Spectroscopy and Instruments

NMR spectra were recorded on a Bruker Ascend^{™} 500 spectrometer operating at 500 MHz, 471 MHz and 126 MHz, for ¹H, ¹⁹F and ¹³C acquisitions, respectively; or on a Varian Unity/lnova 600 spectrometer operating at 600 MHz and 151 MHz for ¹H and ¹³C acquisitions, respectively; or on a Bruker Ultrashield^{™} 300 spectrometer operating at 300 MHz, 282 MHz and 75 MHz for ¹H, ¹⁹F and ¹³C acquisitions, respectively. Chemical shifts are reported in ppm with the solvent residual peak as the internal standard. For ¹H NMR: CDCl₃, δ 7.26; CD₃CN, δ 1.96; CD₂Cl₂, δ 5.32; For ¹³C NMR: CDCl₃, δ 77.16; CD₃CN, δ 1.32; CD₂Cl₂, δ 53.84. ¹⁹F NMR spectra were referenced using a unified chemical shift scale based on the ¹H resonance of tetramethylsilane (1% v/v solution in the respective solvent). Data is reported as follows: s = singlet, d = doublet, t = triplet, q = quartet, quin = quintet, sext = sextet, sept = septet, m = multiplet, bs = broad singlet; coupling constants in Hz; integration.

### Starting materials

Chemicals were used as received from commercial suppliers, unless otherwise stated. Thianthrene *S*-oxide, *N*-tosyl DL-serine methyl ester, 4-methyl-*N*-(3-oxopropyl)benzenesulfonamide (tosylation of 3-amino-1-propanol, then oxidation), *N*-tosyl DL-phenylglycine ethyl ester, dibenzothiophene-S-oxide, and N-Boc-carvedilol were synthesized according to literature reports. Zinc triflate was dried in a desiccator over P₂O₅.

### Experimental Data

### Preparation of vinyl TT⁺ 1

### Preparation of 1 from ethylene

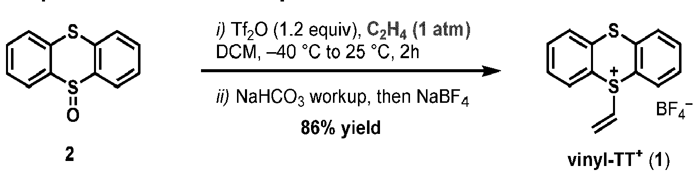

To a round-bottom flask equipped with a stirring bar were added thianthrene-S-oxide 2 (11.7 g, 50.3 mmol, 1.00 equiv.) and 400 mL of DCM (c= 0.125 M). The flask was capped with a rubber septum and cooled down to -40°C. Through the solution was then bubbled ethylene gas for 15 minutes, after which a balloon filled with ethylene was connected to the flask to maintain the ethylene atmosphere throughout the reaction. Triflic anhydride (10.2 mL, 17.0 g, 60.4 mmol, 1.20 equiv.) was added dropwise to the reaction, and a dark purple suspension was progressively formed *[Note: in large scale experiments the amount of precipitate formed can complicate an appropriate stirring. Additional portions of DCM may be added to aid stirring].* After 20 minutes, the cooling bath was removed and the mixture was stirred for 1.5 hour at 25 °C. The ethylene balloon and the rubber septum were removed, and sat. aqueous NaHCO₃ (400 mL) was added carefully. The mixture was vigorously shaken in a separation funnel, phases were separated and the aqueous layer was extracted with DCM (2 × 200 mL). All organic phases were combined, partially concentrated (∼300 mL), washed with aqueous solutions of 5% NaBF₄ (3 × 100 mL), dried over MgSO₄, filtered, and the solvent evaporated under reduced pressure. The crude material was dissolved in DCM (60 mL), and Et₂O (300 mL) was subsequently added while stirring, causing the precipitation of a solid that was collected by filtration, washed with Et₂O (3 × 20 mL), and dried under vacuum to afford 1 as an off-white solid (14.37 g, 43.52 mmol, 86%).

### Preparation of [4+2] cycloadduct intermediate 3

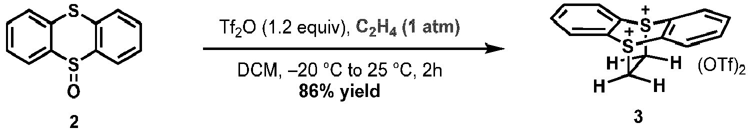

To a round-bottom flask equipped with a stirring bar were added thianthrene-S-oxide 2 (232 mg, 1.00 mmol, 1.00 equiv.) and 8.0 mL of DCM (c= 0.13 M). The flask was capped with a rubber septum and cooled down to -40°C. Through the solution was then bubbled ethylene gas for 5 minutes, after which a balloon filled with ethylene was connected to the flask to maintain the ethylene atmosphere throughout the reaction. Triflic anhydride (202 µL, 338 mg, 1.20 mmol, 1.20 equiv.) was added dropwise to the reaction and a dark purple suspension was progressively formed. After 20 minutes the cooling bath was removed, and the mixture was stirred for 1.5 hour at 25 °C. The ethylene balloon and the rubber septum were removed, and Et₂O (20 mL) was added. The resultant precipitate was collected by filtration, washed with Et₂O (3 × 5 mL), and dried under vacuum to give 3 as a colourless powder (467 mg, 0.861 mmol, 86%).

### Comparative Reactions of other sulfoxides with ethylene

### Reaction of diphenylsulfoxide with ethylene

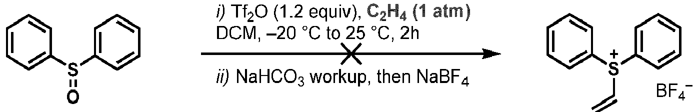

To a round-bottom flask equipped with a stirring bar were added diphenylsulfoxide (101 mg, 0.500 mmol, 1.00 equiv.) and 4 mL of DCM (c= 0.125 M). The flask was capped with a rubber septum and cooled down to -40°C. Through the solution was then bubbled ethylene gas for 5 minutes, after which a balloon filled with ethylene was connected to the flask to maintain the ethylene atmosphere throughout the reaction. Triflic anhydride (101 µL, 169 mg, 0.600 mmol, 1.20 equiv.) was added dropwise to the reaction. After 20 minutes the cooling bath was removed and the mixture was stirred for 1.5 hour at 25 °C. The ethylene balloon and the rubber septum were removed, and solution was concentrated under reduced pressure, diluted with DCM (20 mL) and washed with sat. aqueous NaHCO₃ (20 mL). The aqueous layer was extracted with DCM (2 × 10 mL). All organic phases were combined, washed with aqueous solutions of 5% NaBF₄ (2 × 10 mL), dried over MgSO₄, filtered, and the solvent evaporated to dryness under reduced pressure. CDCl₃ (1 mL) was then added and the crude was analyzed by ¹H NMR, but no vinyl-SPh₂⁺ could be detected.

### Reaction of dibenzothiophene-S-oxide with ethylene

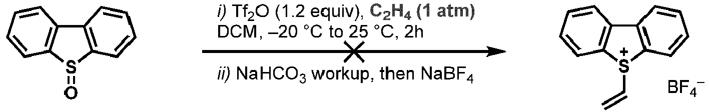

To a round-bottom flask equipped with a stirring bar were added dibenzothiophene-S-oxide (100 mg, 0.500 mmol, 1.00 equiv.) and 4 mL of DCM (c= 0.125 M). The flask was capped with a rubber septum and cooled down to -40°C. Through the solution was then bubbled ethylene gas for 5 minutes, after which a balloon filled with ethylene was connected to the flask to maintain the ethylene atmosphere throughout the reaction. Triflic anhydride (101 µL, 169 mg, 0.600 mmol, 1.20 equiv.) was added dropwise to the reaction. After 20 minutes the cooling bath was removed and the mixture was stirred for 1.5 hour at 25 °C. The ethylene balloon and the rubber septum were removed, and solution was concentrated under reduced pressure, diluted with DCM (20 mL) and washed with aqueous NaHCO₃ (20 mL). The aqueous layer was extracted with DCM (2 × 10 mL). All organic phases were combined, washed with aqueous solutions of 5% NaBF₄ (2 × 10 mL), dried over MgSO₄, filtered, and the solvent evaporated to dryness under reduced pressure. CDCl₃ (1 mL) was then added and the crude was analyzed by ¹H NMR, but no S-vinyl-sulfonium salt could be detected.

### Preparation of vinyl-SPh₂(OTf) (S3)

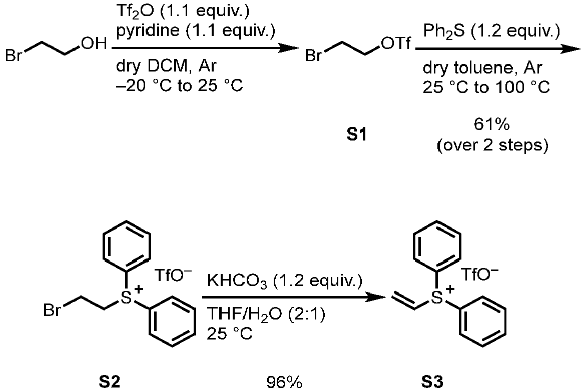

This compound was prepared following the 3-step procedure according to the state of art. To an oven-dried 100 mL round-bottom flask under argon atmosphere containing a teflon-coated magnetic stirring bar were added pyridine (1.7 g, 1.7 mL, 22 mmol, 1.1 equiv.) and anhydrous DCM (35 mL) and the mixture was cooled down to -20 °C. Trifluoromethanesulfonic anhydride (5.9 g, 3.5 mL, 21 mmol, 1.1 equiv.) was added dropwise and the reaction mixture was allowed to stir for 10 minutes at the same temperature. 2-Bromoethanol (2.5 g, 1.4 mL, 20 mmol, 1.0 equiv.) was then added dropwise to the reaction mixture under -20 °C. The cooling bath was removed and the reaction stirred for a further 10 minutes (do not allow more than this time) while warming. The resulting suspension was filtered, concentrated (using a rotary evaporator, keeping the water bath temp below 20 °C) and pentane (30 mL) was added. The mixture was filtered and the filtrate was concentrated again under reduced pressure and dried under vacuum to give the title product **S1** as a clear colorless oil (4.6 g, 89%). It was used immediately in the next step without further purification.

To a round-bottom flask under argon atmosphere containing a teflon-coated magnetic stirring bar were added **S1** (4.58 g, 17.8 mmol, 1.00 equiv.), anhydrous toluene (20 mL) and diphenyl sulfide (4.0 g, 3.6 mL, 15 mmol, 1.2 equiv.) at 25 °C. The reaction mixture was then heated at 100 °C under argon for 5 h. The solution was allowed to cool to 25 °C and diethyl ether (20 mL) was added. The resulting mixture was filtered and the residue was washed with diethyl ether (10 mL) to afford 5.4 g of the title compound **S2** (69% yield) as a white power.

Under ambient atmosphere, a 20 mL vail equipped with a teflon-coated magnetic stirring bar was charged with **S2** (443 mg, 1.00 mmol, 1.00 equiv.) and THF/H₂O (2:1) (3 mL). KHCO₃ (120 mg, 1.20 mmol, 1.20 equiv.) was added in one portion and the reaction mixture was stirred for 30 min at 25 °C (do not allow more than this time). Water (1 mL) was added and the mixture was extracted with DCM (3 × 5 mL). The organic layers were collected, dried over anhydrous Na₂SO₄, filtered and concentrated under reduced pressure. The residue was purified by column chromatography on silica gel eluting with a solvent mixture of methonal:DCM (1:15 (v:v)) to afford 348 mg of the title compound (S3) as a yellow oil (96% yield).

### Annulation reactions employing vinyl-TT⁺ 1

### Spiro[cyclopropane-1,3'-indolin]-2'-one (5)

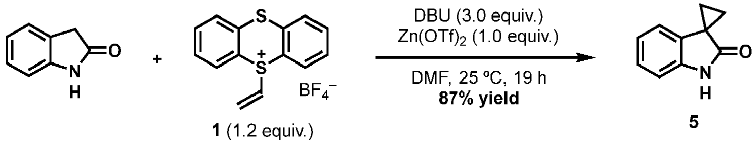

Following a modified reported procedure,^{[9]} 2-oxindole (26.6 mg, 0.200 mmol, 1.00 equiv.), **1** (79.2 mg, 0.240 mmol, 1.20 equiv.), and zinc triflate (72.7 mg, 0.200 mmol, 1.00 equiv.) were dissolved in DMF (1.0 mL, c= 0.20 M) under ambient atmosphere. To this solution was added DBU (90 µL, 92 mg, 0.60 mmol, 3.0 equiv.). After stirring for 19 h at 25 °C, a saturated aqueous solution of NH₄Cl (7 mL) was added, and the phases were separated. The aqueous phase was extracted with EtOAc (3 × 50 mL). All organic phases were combined, washed with water (2 × 10 mL), dried over Na₂SO₄, filtered, and concentrated under reduced pressure. Purification of the residue by column chromatography on silica gel eluting with hexanes/EtOAc (3:1, v/v) afforded the title compound as a pale-yellow solid (27.6 mg, 0.173 mmol, 87%).
**R*f*** = 0.40 (hexanes/EtOAc, 1:1).

### Methyl (±)-4-tosylmorpholine-3-carboxylate (7)

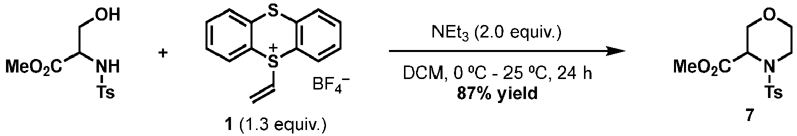

Following a modified reported procedure,^{[2]} *N*-tosyl DL-serine methyl ester (54.7 mg, 0.200 mmol, 1.00 equiv.) was dissolved in dry DCM (1.0 mL, c= 0.20 M) under an argon atmosphere, and the resulting solution was cooled to 0 °C. To the solution was added dry NEt₃ (56 µL, 41 mg, 0.40 mmol, 2.0 equiv.), and after 10 min a solution of 1 (87.4 mg, 0.265 mmol, 1.32 equiv.) in dry DCM (0.5 mL) was added dropwise. After stirring for 3 h at 0 °C, and then for 21 h at 25 °C, a saturated aqueous solution of NH₄Cl (3 mL) was added. The phases were separated, and the aqueous phase was extracted with DCM (3 × 20 mL). All organic phases were combined, washed with brine (20 mL), dried over MgSO₄, filtered, and concentrated under reduced pressure. Purification of the residue by column chromatography on silica gel eluting with hexanes/EtOAc (3:1 to 1:1, v/v) afforded the title compound as a colorless solid (52.2 mg, 0.174 mmol, 87%). The NMR spectra are in good accordance with the literature.
**R*f*** = 0.54 (hexanes/EtOAc).

### Ethyl (±)-2-phenyl-1-tosylazetidine-2-carboxylate (9)

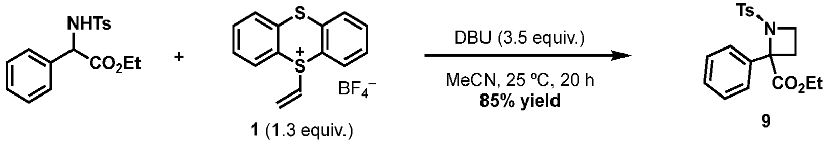

Following a modified reported procedure, a vial was charged with *N*-tosyl DL-phenylglycine ethyl ester (55.7 mg, 0.167 mmol, 1.00 equiv.), 1 (69.3 mg, 0.210 mmol, 1.26 equiv.), and MeCN (2.3 mL, c= 0.073 M) at 25 °C under air. Then, DBU (87 µL, 89 mg, 0.58 mmol, 3.5 equiv.) was added. The reaction was stirred for 20 h at 25 °C and was then concentrated under reduced pressure. The residue was purified by column chromatography on silica gel eluting with pentane/EtOAc (100% pentane to 5:1, v/v). The title compound (51.2 mg, 0.142 mmol, 85%) was obtained as a colorless oil.
**R*f*** = 0.21 (pentane/EtOAc, 5:1).

### (±)-3-Tosyl-7-oxa-3-azabicyclo[4.1.0]heptane (11)

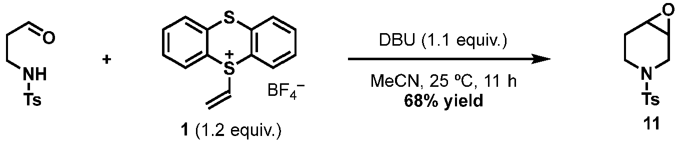

Epoxide **11** was prepared following a modified reported procedure.^{[11]} Under air, DBU (14 µL, 14 mg, 0.094 mmol, 1.1 equiv.) was added to a mixture of 4-methyl-*N*-(3-oxopropyl)benzenesulfonamide^{[4]} (19.0 mg, 0.0836 mmol, 1.00 equiv.) (used immediately after preparation) and **1** (33.1 mg, 0.100 mmol, 1.20 equiv.) in MeCN (1.0 mL, c= 0.084 M). The reaction mixture was stirred at 25 °C for 11 h and was then concentrated under reduced pressure. Purification of the residue by column chromatography on silica gel eluting with hexanes/EtOAc (3:1, v/v) afforded the title compound as a colorless solid (14.3 mg, 0.0565 mmol, 68%).
**R*f*** = 0.48 (hexanes/EtOAc, 1:1).

### Vinylation of N-heterocycles using vinyl-TT⁺

### General procedure A

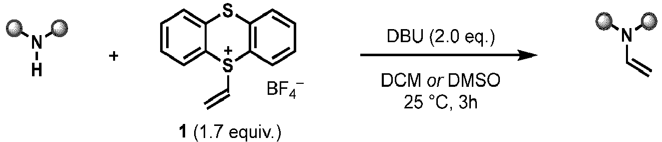

Under air, a vial was charged with the substrate to be vinylated (0.300 mmol, 1.00 equiv.) and 1 (168 mg, 0.510 mmol, 1.70 equiv.). DCM or DMSO (3.0 mL, c= 0.10 M) was added, followed by DBU (90 µL, 92 mg, 0.60 mmol, 2.0 equiv.), and the mixture was stirred at 25 °C for 3 h. Then, the solvent was removed under reduced pressure, and the residue was purified as indicated to give the corresponding product.

### 9-Vinyl-9H-pyrido[3,4-b]indole (12)

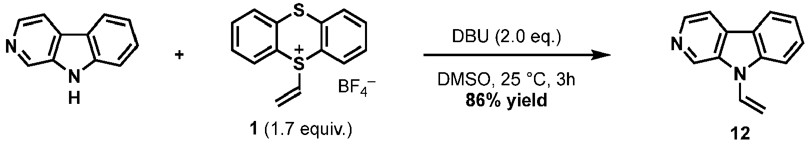

The title compound was prepared following general procedure A. Under air, a vial was charged with norharmane (50.5 mg, 0.300 mmol, 1.00 equiv.) and **1** (119 mg, 0.360 mmol, 1.20 equiv.). DMSO (3.0 mL, *c*= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with hexanes/EtOAc (100% hexanes to 1:1, containing 1% of NEt₃) yielded 9-vinyl-9*H*-pyrido[3,4-*b*]indole **(12)** as a yellow oil (50.0 mg, 0.257 mmol, 86%).
**R*f*** = 0.22 (hexanes/EtOAc, 1:1).

### 5-Nitro-1-vinyl-1H-indole (13)

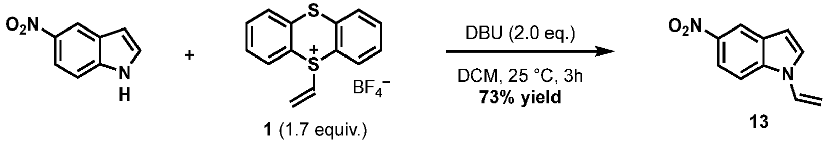

The title compound was prepared following general procedure A. Under air, a vial was charged with 5-nitroindole (48.6 mg, 0.300 mmol, 1.00 equiv.) and **1** (168 mg, 0.510 mmol, 1.70 equiv.). DCM (3.0 mL, *c*= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with hexanes/EtOAc (100% hexanes to 10:1, containing 1% of NEt₃) yielded 5-nitro-1-vinyl-1*H*-indole **(13)** as a yellow solid (41.3 mg, 0.219 mmol, 73%).
**R*f*** = 0.37 (hexanes/EtOAc, 4:1).

### 1-Vinyl-1H-indole-3-carbaidehyde (14)

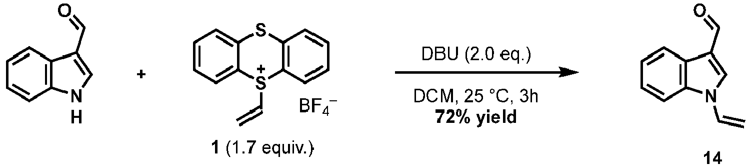

The title compound was prepared following general procedure A. Under air, a vial was charged with 1*H*-indole-3-carbaldehyde (43.5 mg, 0.300 mmol, 1.00 equiv.) and **1** (168 mg, 0.510 mmol, 1.70 equiv.). DCM (3.0 mL, c= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with DCM/EtOAc (100% DCM to 4:1, containing 1% of NEt₃) yielded 1-vinyl-1*H*-indole-3-carbaldehyde (**14**) as a pale-yellow oil (37.2 mg, 0.217 mmol, 72%).
**R*f*** = 0.20 (DCM).

### Ethyl 3-amino-1-vinyl-1H-pyrazole-4-carboxylate (15-I) and ethyl 5-amino-1-vinyl-1H-pyrazole-4-carboxylate (15-II)

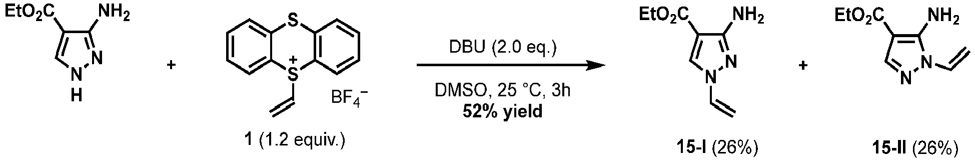

Under air, a vial was charged with ethyl 3-amino-1H-pyrazole-4-carboxylate (46.5 mg, 0.300 mmol, 1.00 equiv.) and DMSO (2.0 mL). DBU (90 µL, 92 mg, 0.60 mmol, 2.0 equiv.) was added, and the mixture was stirred for 5 min at 25 °C. Then, a solution of **1** (119 mg, 0.360 mmol, 1.20 equiv.) in DMSO (1.0 mL) was added, and the resulting solution was stirred at 25 °C for 3 hours. The mixture was then diluted with DCM (20 mL) and washed with H₂O (20 mL). The aqueous phase was extracted with DCM (2 × 10 mL) and the combined organic phases were washed with brine (20 mL), dried over MgSO4 and the solvent removed under reduced pressure. Purification by column chromatography silica gel eluting with hexanes/EtOAc (8:1 to 3:1, containing 1% of NEt₃) yielded ethyl 3-amino-1-vinyl-1H-pyrazole-4-carboxylate (**15-I**) (14.0 mg, 0.077 mmol, 26%) and ethyl 5-amino-1-vinyl-1H-pyrazole-4-carboxylate (**15-II**) (14.0 mg, 0.077 mmol, 26%), both as a colorless solids.

### Data for 15-I:

**R*f*** = 0.36 (hexanes/EtOAc, 3:1).

### 4-Bromo-3,5-dimethyl-1-vinyl-1H-pyrazole (16)

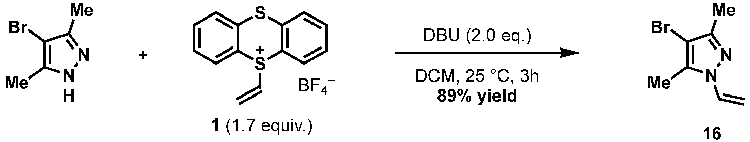

The title compound was prepared following general procedure A. Under air, a vial was charged with 4-bromo-3,5-dimethyl-1*H*-pyrazole (52.5 mg, 0.300 mmol, 1.00 equiv.) and **1** (168 mg, 0.510 mmol, 1.70 equiv.). DCM (3.0 mL, c= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with DCM (containing 1% of NEt₃) yielded 4-bromo-3,5-dimethyl-1-vinyl-1*H*-pyrazole **(16)** as a colorless oil (53.5 mg, 0.266 mmol, 89%).
**R*f*** = 0.30 (DCM).

### Methyl 1-vinyl-1H-1,2,4-triazole-3-carboxylate (17)

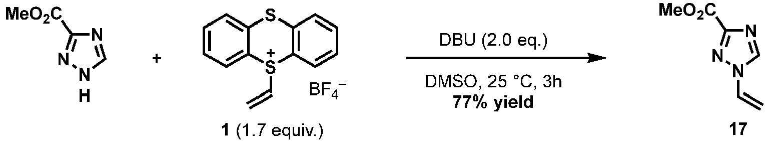

The title compound was prepared following general procedure A. Under air, a vial was charged with methyl 4*H*-1,2,4-triazole-3-carboxylate (38.1 mg, 0.300 mmol, 1.00 equiv.) and 1 (168 mg, 0.510 mmol, 1.70 equiv.). DMSO (3.0 mL, c= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with DCM/EtOAc (100% DCM to 2:1, containing 1% of NEt₃) yielded methyl 1-vinyl-1*H*-1,2,4-triazole-3-carboxylate **(17)** as an off-white solid (35.3 mg, 0.231 mmol, 77%).
**R*f*** = 0.27 (DCM/EtOAc, 2:1).

### 1-Vinylpyridin-4(1H)-one (18)

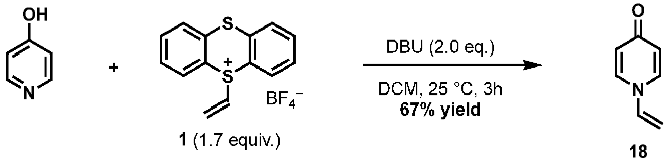

The title compound was prepared following general procedure A. Under air, a vial was charged with 4-hydroxypyridine (28.5 mg, 0.300 mmol, 1.00 equiv.) and **1** (168 mg, 0.510 mmol, 1.70 equiv.). DCM (3.0 mL, c= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with DCM/MeOH (50:1 to 20:1, containing 1% of NEt₃) yielded 1-vinylpyridin-4(1*H*)-one (**18**) as a colorless solid (24.3 mg, 0.201 mmol, 67%).
**R*f*** = 0.08 (DCM/MeOH, 20:1).

### 4-(4-Fluorophenyl)-1-vinyl-1H-imidazole (19)

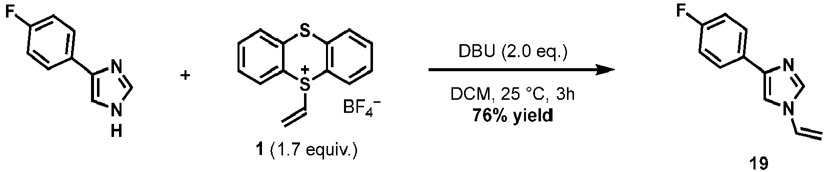

The title compound was prepared following general procedure A. Under air, a vial was charged with 4-(4-fluorophenyl)-1*H*-imidazole (48.6 mg, 0.300 mmol, 1.00 equiv.) and **1** (168 mg, 0.510 mmol, 1.70 equiv.). DCM (3.0 mL, c= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with hexanes/EtOAc (100% hexanes to 1:1, containing 1% of NEt₃) yielded 4-(4-fluorophenyl)-1-vinyl-1*H*-imidazole (19) as a colorless solid (43.0 mg, 0.228 mmol, 76%).
**R*f*** = 0.27 (hexanes/EtOAc, 1:1).

### 4-Chloro-7-vinyl-7H-pyrrolo[2,3-d]pyrimidine (20)

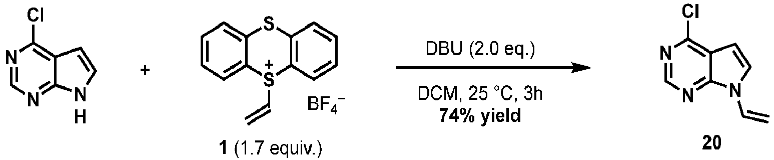

The title compound was prepared following general procedure A. Under air, a vial was charged with 4-chloro-7*H*-pyrrolo[2,3-*d*]pyrimidine (46.1 mg, 0.300 mmol, 1.00 equiv.) and **1** (168 mg, 0.510 mmol, 1.70 equiv.). DCM (3.0 mL, *c*= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with hexanes/EtOAc (100% hexanes to 7:1, containing 1% of NEt₃) yielded 4-chloro-7-vinyl-7*H*-pyrrolo[2,3-*d*]pyrimidine **(20)** as a colorless solid (40.1 mg, 0.223 mmol, 74%).
**R*f*** = 0.40 (hexanes/EtOAc, 4:1).

### N-Vinyl-theophylline (21)

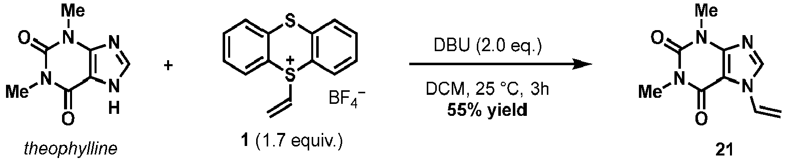

The title compound was prepared following general procedure A. Under air, a vial was charged with theophylline (54.0 mg, 0.300 mmol, 1.00 equiv.) and **1** (168 mg, 0.510 mmol, 1.70 equiv.). DCM (3.0 mL, *c*= 0.10 M) was added, followed by DBU (90 µL, 92 mg, 0.60 mmol, 2.0 equiv.), and the mixture was stirred at 25 °C for 3 h. Then, the solvent was removed under reduced pressure. Purification by column chromatography on silica gel eluting with DCM/EtOAc (100% DCM to 1:1, containing 1% of NEt₃) yielded N-vinyl-theophylline **(21)** as a colorless solid (34.0 mg, 0.165 mmol, 55%).
**R*f*** = 0.19 (DCM/EtOAc, 2:1).

### N-Vinyl,N'-Acetyl-L-tryptophan ethyl ester (22)

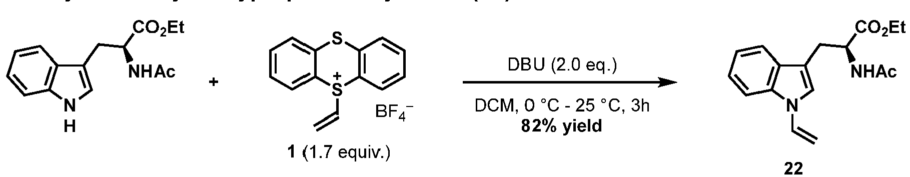

Under air, a vial was charged with *N*-acetyl-L-tryptophan ethyl ester (82.3 mg, 0.300 mmol, 1.00 equiv.) and DCM (3.0 mL, c= 0.10 M). DBU (90 µL, 92 mg, 0.60 mmol, 2.0 equiv.) was added, and the mixture was cooled to 0 °C. At 0 °C, a solution of 1 (168 mg, 0.510 mmol, 1.70 equiv.) in DCM (1.0 mL) was added, and the resulting solution was stirred at 0 °C for 30 min, followed by 2.5 h at 25 °C. After that, the solvent was removed under reduced pressure. Purification by column chromatography on silica gel eluting with DCM/EtOAc (5:1, containing 1% of NEt₃) yielded *N*-vinyl,*N*'-acetyl-L-tryptophan ethyl ester **(22)** as a colorless solid (74.0 mg, 0.246 mmol, 82%).
**R*f*** = 0.16 (DCM/EtOAc, 5:1).

### N-Vinyl,N'-Boc-L-histidine methyl ester (23)

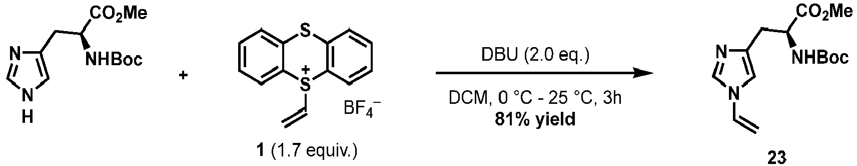

Under air, a vial was charged with *N*-Boc-L-histidine methyl ester (80.8 mg, 0.300 mmol, 1.00 equiv.), **1** (168 mg, 0.510 mmol, 1.70 equiv.) and DCM (3.0 mL, c= 0.10 M), and the mixture was cooled to 0 °C. DBU (90 µL, 92 mg, 0.60 mmol, 2.0 equiv.) was then added and the resulting solution was stirred at 0 °C for 30 min, followed by 2.5 h at 25 °C. After that, the solvent was removed under reduced pressure. Purification by column chromatography on silica gel eluting with DCM/MeOH (100:1 to 20:1, containing 1% of N Et₃) yielded *N*-vinyl,*N*'-Boc-L-histidine methyl ester **(23)** as an off-white solid (72.0 mg, 0.244 mmol, 81%).
**R*f*** = 0.21 (DCM/MeOH, 50:1).

### N-Vinyl-metaxalone (24)

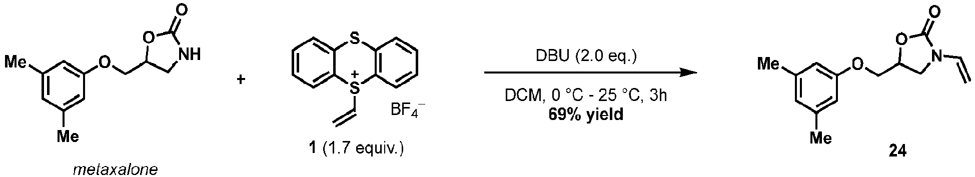

Under air, a vial was charged with metaxalone (66.4 mg, 0.300 mmol, 1.00 equiv.), 1 (168 mg, 0.510 mmol, 1.70 equiv.) and DCM (3.0 mL, *c*= 0.10 M), and the mixture was cooled to 0 °C. DBU (90 µL, 92 mg, 0.60 mmol, 2.0 equiv.) was then added, and the resulting solution was stirred at 0 °C for 30 min, followed by 2.5 h at 25 °C. After that, the solvent was removed under reduced pressure. Purification by column chromatography on silica gel eluting with hexanes/EtOAc (9:1 to 4:1, containing 1% of NEt₃) yielded *N*-vinyl-metaxalone **(24)** as a colorless solid (51 mg, 0.206 mmol, 69%).
**R*f*** = 0.25 (hexanes/EtOAc, 4:1).

### N-Vinyl,N'-Boc-carvedilol (25)

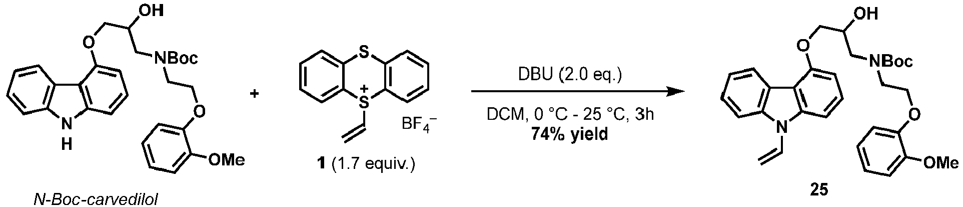

Under air, a vial was charged with N-Boc-carvedilol (151 mg, 0.300 mmol, 1.00 equiv.), 1 (168 mg, 0.510 mmol, 1.70 equiv.) and DCM (3.0 mL, *c*= 0.10 M), and the mixture was cooled to 0 °C. DBU (90 µL, 92 mg, 0.60 mmol, 2.0 equiv.) was then added, and the resulting solution was stirred at 0 °C for 30 min, followed by 2.5 h at 25 °C. After that, the solvent was removed under reduced pressure. Purification by column chromatography on silica gel eluting with DCM/EtOAc (100:0 to 20:1, containing 1% of NEt₃) yielded *N*-vinyl-*N'-*Boc-carvedilol **(25)** as a colorless solid (118 mg, 0.226 mmol, 74%).
**R*f*** = 0.41 (DCM/EtOAc, 9:1).

### N-Vinyl-coumarin 7 (26)

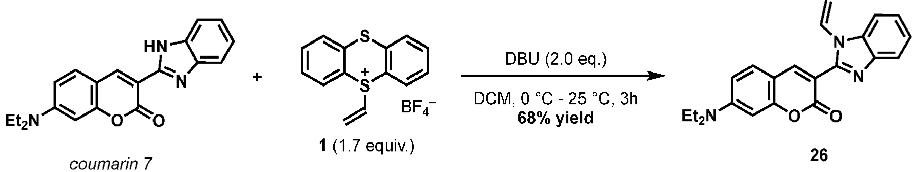

Under air, a vial was charged with coumarin 7 (100 mg, 0.300 mmol, 1.00 equiv.), 1 (168 mg, 0.510 mmol, 1.70 equiv.) and DCM (3.0 mL, *c*= 0.10 M), and the mixture was cooled to 0 °C. DBU (90 µL, 92 mg, 0.60 mmol, 2.0 equiv.) was then added, and the resulting solution was stirred at 0 °C for 30 min, followed by 2.5 h at 25 °C. After that, the solvent was removed under reduced pressure. Purification by column chromatography on silica gel eluting with DCM/EtOAc (100:0 to 20:1, containing 1% of NEt₃) yielded *N*-vinyl-coumarin 7 **(26)** as a bright yellow solid (73 mg, 0.21 mmol, 68%).
**R*f*** = 0.42 (DCM/EtOAc, 9:1).

### N-Vinyl-lansoprazole (27)

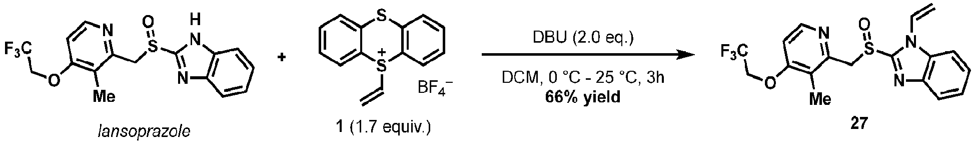

Under air, a vial was charged with lansoprazole (111 mg, 0.300 mmol, 1.00 equiv.) and DCM (3.0 mL). DBU (90 µL, 92 mg, 0.60 mmol, 2.0 equiv.) was added, and the mixture was cooled to 0 °C. At 0 °C, a solution of **1** (168 mg, 0.510 mmol, 1.70 equiv.) in DCM (1.0 mL) was added, and the resulting solution was stirred at 0 °C for 30 min, followed by 2.5 h at 25 °C. After that, the solvent was removed under reduced pressure. Purification by column chromatography on silica gel eluting with DCM/EtOAc (1:2, containing 1% of NEt₃) yielded *N*-vinyl-lansoprazole **(27)** as a colorless solid (78.1 mg, 0.198 mmol, 66%).
**R*f*** = 0.15 (DCM/EtOAc, 1:2).

### Suzuki-type vinylation of aryl organoboron compounds using vinyl-TT⁺ 1

### General procedure B

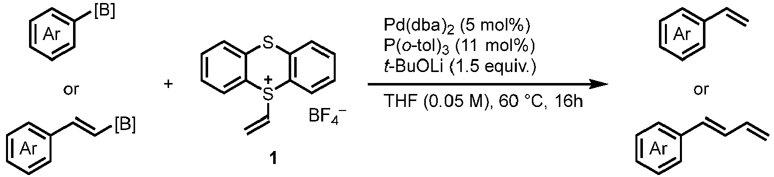

Under ambient atmosphere, a 20 mL vial equipped with a teflon-coated magnetic stirring bar was charged with organoboron species (0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before 1 (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel to give corresponding product. *[Note: unless otherwise mentioned, t-BuOLi stored at ambient atmosphere was used. When extra-dry t-BuOLi stored in the glovebox was used, poor yields were obtained].*

### General procedure for the vinylation of organoboron compounds using a Schlenk line

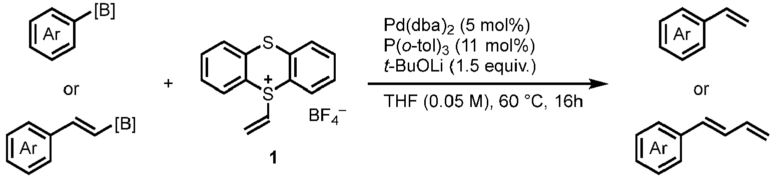

Under ambient atmosphere, a 20 mL Schlenk tube equipped with a teflon-coated magnetic stirring bar was charged with organoboron species (0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The Schlenk tube was evacuated and backfilled with argon. Subsequently, dry THF (6 mL, c = 0.05 M) was added into the Schlenk tube via a syringe. The reaction mixture was stirred for 2 min at 25 °C before **1** (149 mg, 0.450 mmol, 1.50 equiv.) was added into the Schlenk tube in one portion. The Schlenk tube was placed in an oil bath preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel to give corresponding product.

### 1-Methyl-3-vinylbenzene (28)

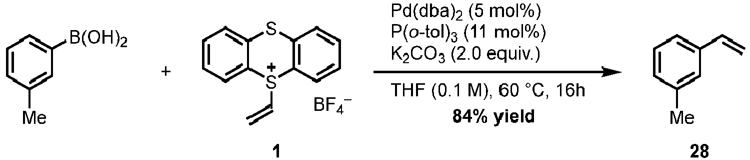

Under ambient atmosphere, a 4 mL vial equipped with a teflon-coated magnetic stirring bar was charged with 3-methylbenzeneboronic acid (6.8 mg, 0.050 mmol, 1.0 equiv.), Pd(dba)₂ (1.4 mg, 2.5 µmol, 5.0 mol%), P(*o*-tol)₃ (1.7 mg, 5.5 µmol, 11 mol%) and *t*-BuOLi (6.0 mg, 0.075 mmol, 1.5 equiv.). The vial was transferred into a N₂-filled glove box. Subsequently, dry THF (0.5 mL, c = 0.1 M) was added into the vial. The reaction mixture was stirred for 2 min at 25 °C before **1** (24.8 mg, 0.0750 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (4 mL). The filtrate was collected and concentrated under reduced pressure. Due to the volatility of the title product, its yield was determined via NMR analysis of the reaction mixture. To the residue was added dibromomethane (7.0 µL, 17 mg, 0.10 mmol, 2.0 equiv.) as an internal standard. TheH NMR resonances of the vinyl protons of the product between 5.6 and 5.8 ppm were integrated relative to the ¹H NMR resonances of the protons of dibromomethane (δ = 4.90 ppm). The yield was determined as 84% (Figure S9).

### 3-Methyl-4-vinylbenzonitrile (29)

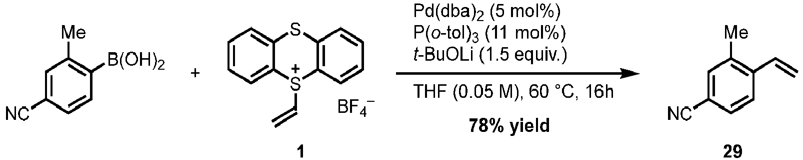

The title compound was prepared following general procedure B. Under ambient atmosphere, a 20 mL vial equipped with a teflon-coated magnetic stirring bar was charged with 2-methyl-4-cyanophenylboronic acid (48.3 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with a solvent mixture of EtOAc:pentane (1:50 (v:v)) to afford 33.5 mg of the title compound **(29)** as a colorless oil (78% yield).
**R*f*** = 0.35 (EtOAc:pentane, 1:19 (v:v)).

### 1-Chloro-4-methoxy-2-vinylbenzene (30)

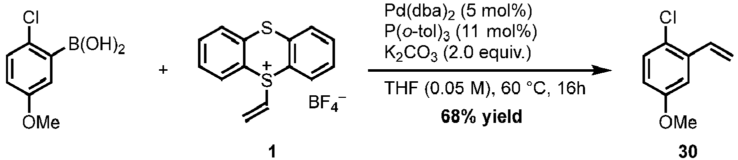

The title compound was prepared following general procedure B. Under ambient atmosphere, a 20 mL vial equipped with a teflon-coated magnetic stirring bar was charged with 2-chloro-5-methoxyphenylboronic acid (55.9 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and K₂CO₃ (82.9 mg, 0.600 mmol, 2.00 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 34.3 mg of the title compound **(30)** as a colorless oil (68% yield).
**R*f*** = 0.40 (EtOAc:pentane, 1:19 (v:v)).

### 1-Chloro-4-vinylbenzene (31)

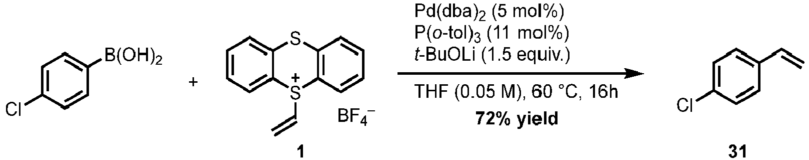

The title compound was prepared following general procedure B. Under ambient atmosphere, a 20 mL vial equipped with a teflon-coated magnetic stirring bar was charged with 4-chlorophenylboronic acid (46.9 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 34.3 mg of the title compound **(31)** as a colorless oil (72% yield).
**R*f*** = 0.51 (pentane).

### 1-Ethoxy-2-vinylbenzene (32)

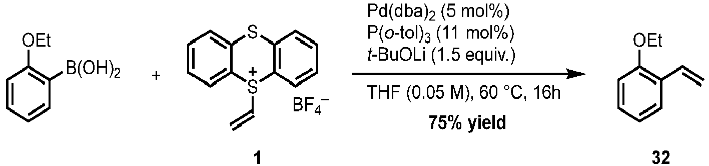

The title compound was prepared following general procedure B. Under ambient atmosphere, a 20 mL vial equipped with a teflon-coated magnetic stirring bar was charged with 2-ethoxyphenylboronic acid (49.8 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 33.3 mg of the title compound **(32)** as a colorless oil (75% yield).
**R*f*** = 0.51 (EtOAc:pentane, 1:19 (v:v)).

### 1-Bromo-4-vinylbenzene (33)

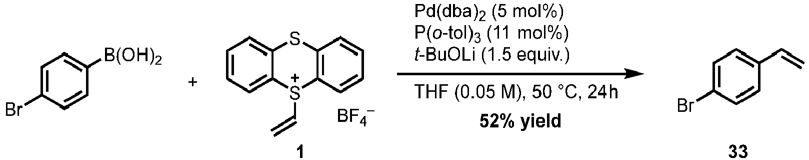

Under ambient atmosphere, a 20 mL vial equipped with a teflon-coated magnetic stirring bar was charged with **1** (168 mg, 0.510 mmol, 1.70 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before 4-bromophenylboronic acid (60.2 mg, 0.300 mmol, 1.00 equiv.) was added into the vial in one portion. The vial was capped and was then transferred out of the glove box. The vial was placed on a heating block preheated at 50 °C where the reaction mixture was stirred for 24 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 28.5 mg of the title compound **(33)** as a colorless oil (52% yield).
**R*f*** = 0.51 (pentane).

### 4-Vinylbenzaldehyde (34)

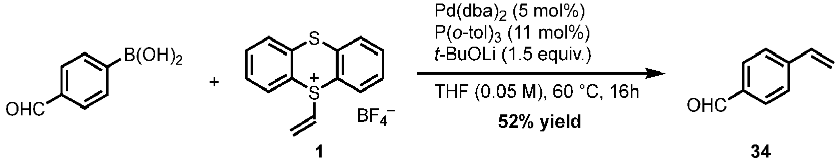

The title compound was prepared following general procedure B. Under ambient atmosphere, a 20 mL vial equipped with a teflon-coated magnetic stirring bar was charged with 4-formylphenylboronic acid (45.0 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with a solvent mixture of EtOAc:pentane (1:50, (v:v)) to afford 21.0 mg of the title compound **(34)** as a colorless oil (52% yield).
**R*f*** = 0.29 (EtOAc:pentane, 1:19 (v:v)).

### 2-Vinylbenzo[b]thiophene (35)

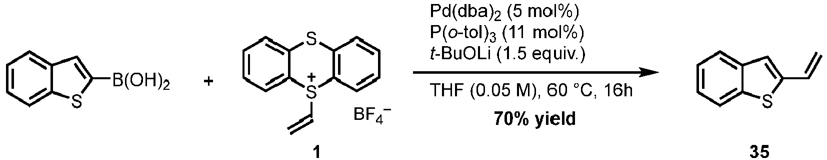

The title compound was prepared following general procedure B. Under ambient atmosphere, a 20 mL vial equipped with a teflon-coated magnetic stirring bar was charged with benzo[b]thien-2-ylboronic acid (53.4 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 33.6 mg of the title compound **(35)** as a white solid (70% yield).
**R*f*** = 0.29 (pentane).

### 1-Methyl-3-(trifluoromethyl)-5-vinyl-1H-pyrazole (36)

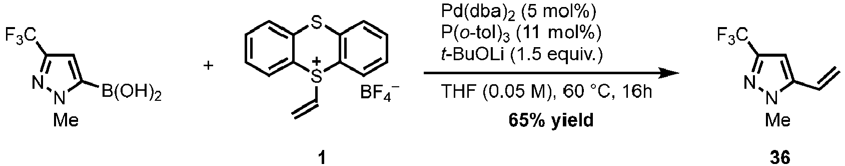

The title compound was prepared following general procedure B. Under ambient atmosphere, a 20 mL vial equipped with a teflon-coated magnetic stirring bar was charged with (1-methyl-3-(trifluoromethyl)-1*H*-pyrazol-5-yl)boronic acid (58.2 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure at temperature < 30 °C. The residue was purified by column chromatography on silica gel eluting with a solvent mixture of DCM:pentane (1:2, (v:v)) to afford 34.3 mg of the title compound **(36)** as a colorless oil (65% yield). *[Note: due to the volatility of the product, drying of the purified product was processed under vacuum in a bath of dry ice].*
**R*f*** = 0.30 (DCM:pentane, 1:1 (v:v)).

### 1-Chloro-4-(trifluoromethyl)-2-((2-vinylbenzyl)oxy)benzene (37)

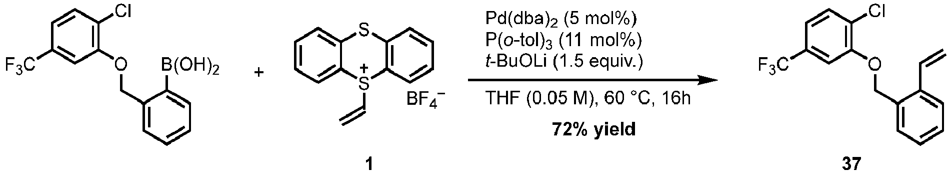

The title compound was prepared following general procedure B. Under ambient atmosphere, a 20 mL vial equipped with a teflon-coated magnetic stirring bar was charged with 2-((2'-Chloro-5'-(trifluoromethyl)phenoxy)methyl)phenylboronic acid (99.1 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 67.4 mg of the title compound **(37)** as a white solid (72% yield).
**R*f*** = 0.51 (EtOAc:pentane, 1:19 (v:v)).

### Piperidin-1-yl(4-vinylphenyl)methanone (38)

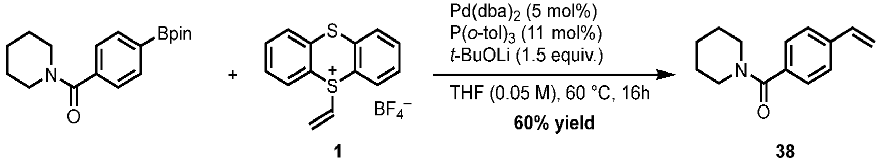

The title compound was prepared following general procedure B. Under ambient atmosphere, a 20 mL vial equipped with a teflon-coated magnetic stirring bar was charged with 4-(piperidine-1-carbonyl)phenylboronic acid pinacol ester (94.6 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with a solvent mixture of EtOAc:pentane (1:4 (v:v)) to afford 38.8 mg of the title compound **(38)** as a white solid (60% yield).
**R*f*** = 0.40 (EtOAc:pentane, 1:1 (v:v)).

### Morpholino(3-vinylphenyl)methanone (39)

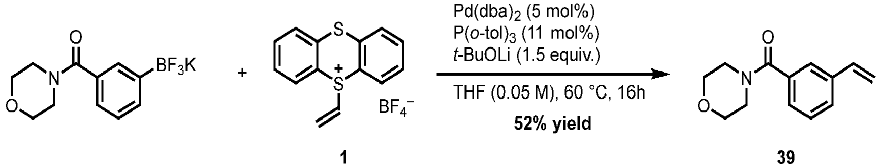

The title compound was prepared following general procedure B. Under ambient atmosphere, a 20 mL vial equipped with a teflon-coated magnetic stirring bar was charged with potassium 3-(4-morpholinylcarbonyl)phenyltrifluoroborate (89.1 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before **1** (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 34.3 mg of the title compound (39) as a colorless oil (52% yield).
**R*f*** = 0.23 (EtOAc:pentane, v:v (1:1)).

### (E)-4-(Buta-1,3-dien-1-yl)-1,1'-biphenyl (40)

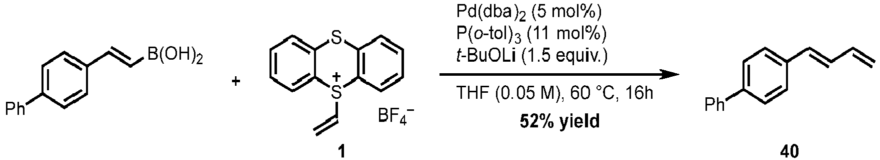

The title compound was prepared following general procedure B. Under ambient atmosphere, a 20 mL vial equipped with a teflon-coated magnetic stirring bar was charged with *trans*-2-(4-Biphenyl)vinylboronic acid (67.2 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (6 mL, c = 0.05 M) was added into the vial. The reaction mixture was stirred for 2 min at 25 °C before 1 (149 mg, 0.450 mmol, 1.50 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with pentane to afford 32.0 mg of the title compound **(40)** as a white solid (52% yield).
**R*f*** = 0.20 (pentane).

### Comparison on the performance of 1 and vinyl-SPh₂(OTf) in Suzuki-type reactions. General procedure C

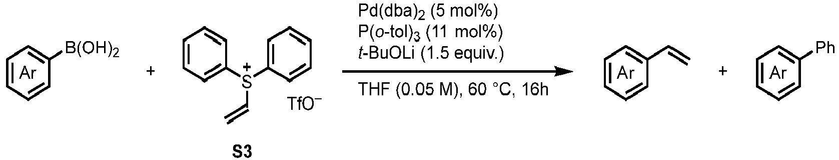

Under ambient atmosphere, a 20 mL vial equipped with a teflon-coated magnetic stirring bar was charged with aryl boronic acid (0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and *t*-BuOLi (36.0 mg, 0.450 mmol, 1.50 equiv.). The vial was transferred into a N₂-filled glove box. Subsequently, dry THF (4 mL) was added into the vial. The reaction mixture was stirred for 2 min at 25 °C before a solution of vinylSPh₂(OTf) (**S3,** 163 mg, 0.450 mmol, 1.50 equiv.) in THF (2 mL) was added into the vial via syringe. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel to give corresponding product.

### tert-Butyl (3-vinylphenyl)carbamate (45)

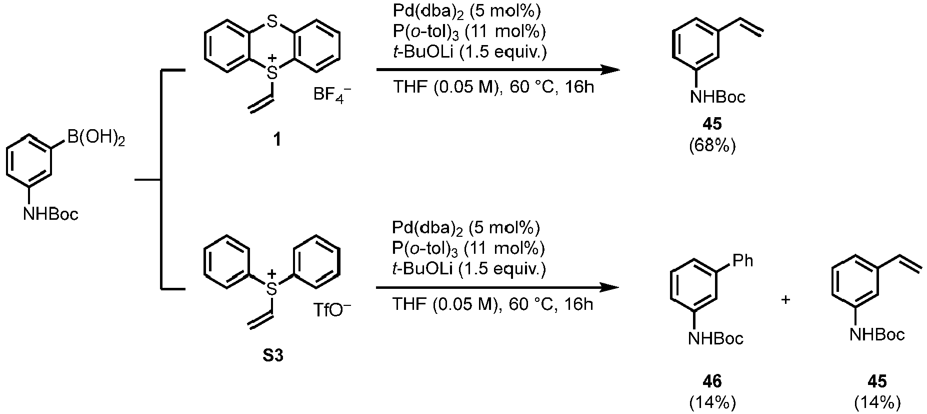

The title compound was prepared following general procedure B (for 1) or C (for S3). Under ambient atmosphere, a 20 mL vial equipped with a teflon-coated magnetic stirring bar was charged with 3-(*N*-Boc-amino)phenylboronic acid (71.1 mg, 0.300 mmol, 1.00 equiv.), Pd(dba)₂ (8.6 mg, 15 µmol, 5.0 mol%), P(*o*-tol)₃ (10.0 mg, 33.0 µmol, 11.0 mol%) and t-BuOLi (36.0 mg, 0.450 mmol, 1.50 equiv.). The vial was transferred into a N₂-filled glove box. Subsequently, dry THF (4 mL) was added into the vial. The reaction mixture was stirred for 2 min at 25 °C before **1** (149 mg, 0.450 mmol, 1.50 equiv.) or **S3** (163 mg, 0.450 mmol, 1.50 equiv; as a solution in 2 mL of THF) were added into the vial in one portion. The vial was capped and was then transferred out of the glove box. The vial was placed on a heating block preheated at 60 °C where the reaction mixture was stirred for 16 h. The reaction mixture was cooled to 25 °C and filtered through a pad of celite eluting with DCM (20 mL). The filtrate was collected and concentrated under vacuum to roughly 5 mL. Silica gel (approximately 300 mg) was added, and the mixture was evaporated to dryness under reduced pressure. The residue was purified by column chromatography on silica gel eluting with a solvent mixture of EtOAc:pentane (1:50 (v:v)) to afford the title compound **(45)** as a colorless oil.

Yield of **45** using **1** as vinylating reagent: 44.7 mg, 68%.

Yield of **45** using **3** as vinylating reagent: 9.3 mg, 14%. In addition, arylation product **46** was obtained as a colorless oil: 11.3 mg, 14% yield.

### Data for 45:

**R*f*** = 0.30 (EtOAc:pentane, 1:19 (v:v)).

### 1-Fluoro-4-vinylbenzene (42)

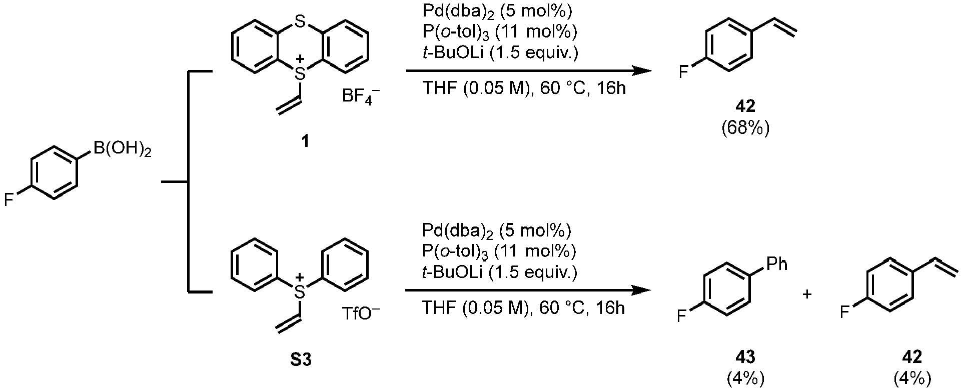

The title compound was prepared following general procedure B (for 1) or C (for **S3).** Under ambient atmosphere, a 4 mL vial equipped with a teflon-coated magnetic stirring bar was charged with (4-fluorophenyl)boronic acid (7.0 mg, 0.050 mmol, 1.0 equiv.), Pd(dba)₂ (1.4 mg, 2.5 µmol, 5.0 mol%), P(*o*-tol)₃ (1.7 mg, 5.5 µmol, 11 mol%) and *t*-BuOLi (6.0 mg, 0.075 mmol, 1.5 equiv.). The vial was transferred into a N₂-filled glove box. Subsequently, dry THF (1 mL) was added into the vial. The reaction mixture was stirred for 2 min at 25 °C before **1** (25 mg, 0.075 mmol, 1.5 equiv.) was added into the vial in one portion. The vial was capped and was then transferred out of the glove box. The vial was placed on a heating block preheated at 60 °C where the reaction mixture was stirred for 16 h. The reaction mixture was cooled to 25 °C. To the cooled reaction mixture was added 4-fluorobenzotrifluoride (12.7 µL, 16.4 mg, 0.10 mmol, 2.0 equiv.) as internal standard. The ¹⁹F NMR resonance of the product at -114.6 ppm was integrated relative to the ¹⁹F NMR resonances of the aromatic fluorine atom of 4-fluorobenzotrifluoride (δ = -107.6 ppm). The scale of using **S3** as vinylating reagent was doubled to 0.10 mmol. In this case, 0.10 mmol (1.0 equiv.) of 4-fluorobenzotrifluoride was added as internal standard.

Yield of **42** using **1** as vinylating reagent: 68%

Yield of **42** using **S3** as vinylating reagent: 4%. In addition, arylation product **43** was obtained in 4% yield.

As detailed above, the inventors have developed a convenient vinyl electrophile reagent that is prepared directly from ethylene gas and can be stored in the presence of air and moisture. The reagent has proven to be an effective vinylating reagent and C2 synthon for the synthesis of carbo- and heterocycles, N-vinylated products, styrenes and dienes. The distinct structural features of thianthrenium salts in comparison with other vinyl sulfonium salts enable both the synthesis from ethylene and its superior performance in cross-coupling reactions. Its one-step synthesis, easy-to-handle features, and robust reactivity make it a valuable and versatile reagent that the inventors believe will find synthetic utility in further organic and transition-metal catalyzed transformations.

## Claims

1. A thianthrene compound of the Formula (I): 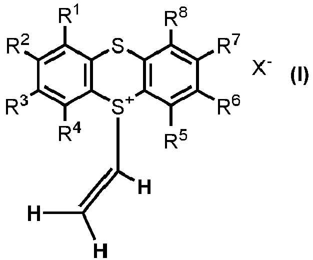 wherein R¹ to R⁸ may be the same or different and are each selected from hydrogen, halogen, -OR⁰ wherein R⁰ is hydrogen or a C₁ to C₆ alkyl group, which may be substituted by at least one halogen, -NR^{N1}R^{N2} wherein R^{N1} and R^{N2} are the same or different and are each hydrogen or a C₁ to C₆ alkyl group, which may be substituted by at least one halogen, or a C₁ to C₆ alkyl group, which may be substituted by at least one halogen, and wherein X⁻ is an anion, selected from F⁻, Cl⁻, triflate⁻ , BF₄⁻, SbF₆⁻, PF₆⁻, BAr₄⁻, TsO⁻, MsO⁻, ClO₄⁻, 0.5 SO₄²⁻, or NO₃⁻ with the proviso that the compound of Formula (I) with R₁ to R₈ being hydrogen and X being ClO₄⁻ is excluded.

2. A thianthrene compound of the Formula (I) as claimed in claim 1 wherein, in Formula (I), R¹ to R⁸ may be the same or different and are each selected from hydrogen, Cl or F and X⁻ is an anion as defined in claim 1, preferably triflate or BF₄⁻, with the proviso that the compound of Formula (I) with R₁ to R₈ being hydrogen and X being ClO₄⁻ is excluded..

3. A thianthrene compound of the Formula (I) as claimed in claim 1 wherein, in Formula (I), R¹ to R⁸ are each hydrogen, and X⁻ is an anion as defined in claim 1, preferably triflate or BF₄⁻, with the proviso that the compound of Formula (I) with R₁ to R₈ being hydrogen and X being ClO₄⁻ is excluded..

4. Process for preparing a vinyl thianthrenium compound of the Formula (I), wherein a thianthrene-S-oxide derivative of the Formula (II) is reacted with ethylene in a closed reaction vessel, at a pressure of at least one atm, in an organic solvent in the presence of triflic acid anhydride or any other combination of an acylating reagent such as trifluoroacetic anhydride in the presence of a Bronsted/Lewis acid, the obtained reaction mixture is treated with an aqueous basic solution and the obtained reaction product is treated with an alkali salt whereby a thianthrenium compound of the Formula (I) is obtained: 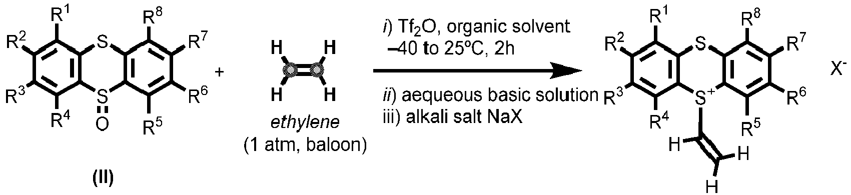 wherein R¹ to R⁸ may be the same or different and are each selected from hydrogen, halogen, -OR⁰ wherein R⁰ is hydrogen or a C₁ to C₆ alkyl group, which may be substituted by at least one halogen, -NR^{N1}R^{N2} wherein R^{N1} and R^{N2} are the same or different and are each hydrogen or a C₁ to C₆ alkyl group, which may be substituted by at least one halogen, or a C₁ to C₆ alkyl group, which may be substituted by at least one halogen, and wherein X⁻ is an anion, selected from F⁻, Cl⁻, triflate⁻, BF₄⁻, SbF₆⁻, PF₆⁻, BAr₄⁻, TsO⁻, MsO⁻, ClO₄⁻, 0.5 SO₄²⁻, or NO₃⁻.

5. Use of a vinyl thianthrenium compound of the Formula (I) wherein R¹ to R⁸ may be the same or different and are each selected from hydrogen, halogen, -OR⁰ wherein R⁰ is hydrogen or a C₁ to C₆ alkyl group, which may be substituted by at least one halogen,, -NR^{N1}R^{N2} wherein R^{N1} and R^{N2} are the same or different and are each hydrogen or a C₁ to C₆ alkyl group, which may be substituted by at least one halogen, or a C₁ to C₆ alkyl group, which may be substituted by at least one halogen, and wherein X⁻ is an anion, selected from F⁻, Cl⁻, triflate⁻, BF₄⁻, SbF₆⁻, PF₆⁻, BAr₄⁻, TsO⁻, MsO⁻, ClO₄⁻, 0.5 SO₄²⁻ as a transfer agent for transferring a vinyl group to a hydrocarbon compound, in particular to an aliphatic hydrocarbon, an aromatic hydrocarbon, a heteroaromatic hydrocarbon, a hydrocarbon bearing at least one nucleophilic heteroatom or an organoboron compound.
